# EUROPEAN PATENT SPECIFICATION

(11) **EP 1 738 738 B1**
(45) Date of publication and mention of the grant of the patent: **11.07.2012**
(21) Application number: 05012065.8
(22) Date of filing: 04.06.2005
(51) Int. Cl.: A61K 8/11, A61K 8/25, A61K 8/73, A61K 8/97, A61K 8/67, A61Q 19/00

(54) **Microcapsules**
Mikrokapseln
Microcapsules

(43) Date of publication of application: 03.01.2007
(73) Proprietor: Cognis IP Management GmbH, 40589 Düsseldorf (DE)
(72) Inventor: Viladot Petit, Josep-Lluis, Dr., 08018 Barcelona (ES); Rathjens, Andreas, Dr., 54510 Tomblaine (FR); De Moragas, Maria, Dr., 08310 Argentona (Barcelona) (ES)
(74) Representative: Fabry, Bernd

(56) References cited:
- US-A- 3 467 544
- US-A- 4 464 317
- US-A- 6 030 645
- DATABASE WPI Section Ch, Week 200148 Derwent Publications Ltd., London, GB; Class B07, AN 2001-449249 XP002351292 & KR 2001 003 163 A (LEE D H) 15 January 2001 (2001-01-15)
- PATENT ABSTRACTS OF JAPAN vol. 016, no. 069 (M-1212), 20 February 1992 (1992-02-20) & JP 03 258585 A (MITSUBISHI PAPER MILLS LTD), 18 November 1991 (1991-11-18)

## Description

### Field of invention

The present invention relates to the area of skin care compositions and claims encapsulated exfoliating agents, cosmetic compositions comprising said capsules and the use of said encapsulated agents for making cosmetic compositions, particular for skin care products.

### State of the art

The cosmetic and pharmaceutical industry is continually expanding its efforts in order to provide topical preparations which will moisten and soften the skin, eliminate dryness, cracking, chapping, chaffing, redness and restore the skin to its natural healthy condition. In addition various cosmetic compositions have been proposed in the prior art to function as skin cleansers or scrubbing agents. For example, US 5,658,573 disclose a body care composition as a facial cleanser which contains colloidal silica particles in an aqueous suspension. Another example of a facial scrub is disclosed in US 5,139,782**,** which discloses a cosmetic composition containing a high-silica zeolite, for removing retention products from a skin surface. While many cosmetic preparations or compositions include inorganic materials for cleansing purposes, a demand exists for all-organic compositions function as cleansers for skin scrubbing agents or masks. Some cosmetic formulations use organic agents such as walnut hulls, almond meal and corn meal.

Drawbacks with these prior art compositions include the use of water as the base material. Water has no therapeutic effect and its inclusion in these prior art compositions merely dilutes the active components. Compositions containing scrubbing agents can be abrasive on the skin, causing redness or throbbing after use. Compositions containing food products such as walnut hulls require the addition of preservatives to prevent the food products from spoiling over time. The preservatives contribute to the cost of the product and further dilute the active components.

In view of the drawbacks noted above, a need exists for improved cosmetic scrubbing compositions, which provides also care effects to the skin.

### Description of the invention

The present invention claims microcapsules having a mean diameter of from about 0.1 to about 5 mm, a membrane and/or a matrix containing at least one active principle, which are characterised in that said active principle is an exfoliating agent, especially an extract of bamboo.

Surprisingly it has been found that microcapsules comprising exfoliating agents which are rich in abrasives, especially bamboo extract as an active principle solve the problem as outlined above in a perfect manner. The capsules can be dried and added to any kind of cosmetic, particularly skin care composition without introducing water, the products are mild to skin and do not need the addition of preservatives (although they can be added if necessary for other purposes). As a matter of fact, the bamboo extracts do not only cause a scrubbing or peeling effect due to the presence of silicates, it has also been found that the other ingredients act as balms and show an anti-inflammatory effect, which is increased in case chitosan is uses as the cationic polymer for encapsulation. These positive effects to skin can be increased by adding further plant extracts and/or pigments. Especially the combination of bamboo and White or Green Tea extract with mica has been found rather advantageous.

Consequently a preferred embodiment of the present invention is directed to microcapsules comprising mixtures of
(a) exfoliating agents, and
(b1) plant extracts and/or
(b2) pigments and/or
(b3) vitamins
as the active principle.

### Exfoliating agents

Exfoliating agents (component a), which can be used according to the present inventions, are those solid compounds having abrasive properties. The preferred components are silicates like rhyolite, lithothamnium or nelumbo nucifera (obtained from the lotos plant), however also powdered avocado kernels or lemon or orange peel can be used. In a preferred embodiment plant extracts rich in silicates are used. Particularly, bamboo extract (*Bambusa spp.*) is well known for its skin-lightening properties and as skin exfoliation promoting compound. Other applications are: oral care, hair tonic, hair growth promoting agent, germicide, and moisture retention improving agent, anti-ageing agent, anti-acne agent, and anti-pruritic agent. In this context reference is made to WO 04/017934 A1 (Coty), which refers to cosmetic compositions having skin-smoothing activity. More particularly, the composition comprises plant extracts selected from i.e. *Bambusa vulgaris.* The application, however, is totally silent with respect to encapsulated plant extracts. Extracts of bamboo are rich in inorganic silicates.

### Plant extracts

Typically, the plant extracts which form component (b1) according to the present invention are chosen from the plants selected from the group consisting of *Ginkgo biloba, Camellia sinensis* (Green Tea or White Tea) *Oleacea europensis, Glyzyrrhiza glabra, Vaccinium myrtillus, Trifolium pratense, Litchi sinensis, Vitis, vinifera, Brassica oleracea, Punica granatum, Petroselinium crispum, Centella asiatica, Passiflora incarnata, Medicago sativa, Valeriana officinalis, Castanea sativa, Salix alba, Hapagophytum procumbens, Aloe barbadensis, Melissa officinalis* and *Citrus Aurantium.* In the following a short summary of the composition and the main constituents of the cited extracts is given.

### • Ginkgo biloba

The active ingredients of extracts from the leaves of the ginkgo tree *(Ginkgo biloba)* are flavonoid glycosides, which among others contain (iso)quercitin glycosides, kaempferol, kaempferol-3-rhamnosides, isorhamnetin, luteoline glycosides, sitosterol glycosides and predominantly hexacyclic terpene lactones, consisting of ginkgolides A, B, C, J, M and bilobalides. 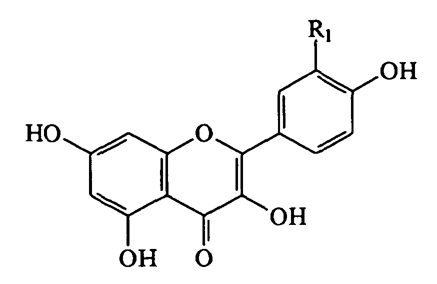 Isorhamnetin (R¹ = H), Kaempferol (R¹ = OH), Ginkgolid A (R¹ = OMe)

### • Camellia sinensis

Leaves of green tea contain many compounds, such as polysaccharides, volatile oils, vitamins, minerals, purines, alkaloids (e.g. caffeine) and polyphenols (catechins and flavonoids). Although all all tea types have antibacterial and free radical capturing (antioxidising) activities, the efficacy decreases substantially the darker the variety of tea is. This is due to the lower contents of anti-oxidising polyphenols remaining in the leaves. Beside extracts of the green leaves of tea, one can also use so-called "white tea" extracts, which does not refer to a special variety of tea, but to a stage of fermentation of green tea leaves. Among the various components of green tea extracts, polyphenols of the flavonoid and catechin type ("tea tannins") are the most important.

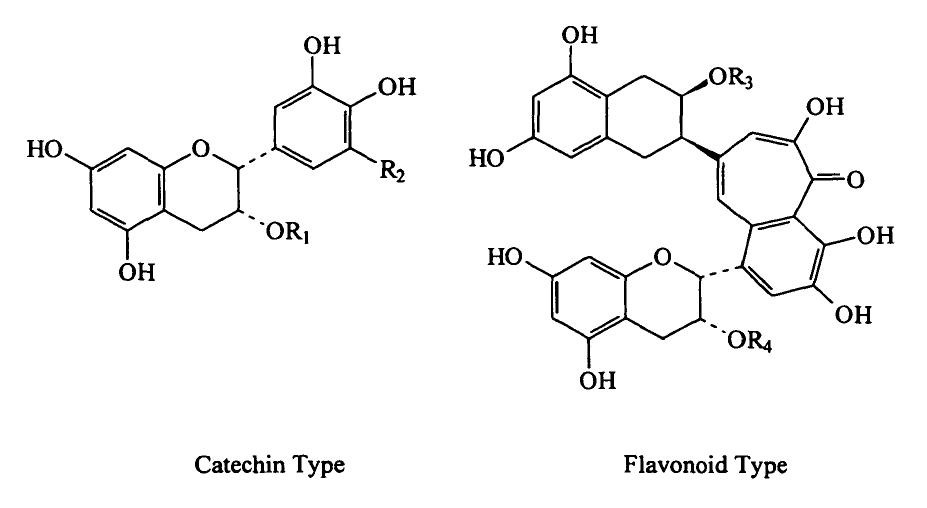

| | R1 | R2 | R3 | R4 |
|---|---|---|---|---|
| (-)-Epicatechin | H | H | | |
| (-) Epigallocatechin | H | OH | | |
| (-) Epicatechin gallate | Galloyl | H | | |
| (-) Epigallocatechin gallate | Galloyl | OH | | |
| Theflavin | | | H | H |
| Theaflavin monogallate A | | | Galloyl | H |
| Theaflavin monogallat B | | | H | Galloyl |
| Theaflavin digallate | | | Galloyl | Galloyl |

### • Oleacea europensis

The main constituent of the leaves of the olive tree (*Oleacea europensis*) is the antioxidant oleuropein, which is also the main source for hydroxytyrosol.

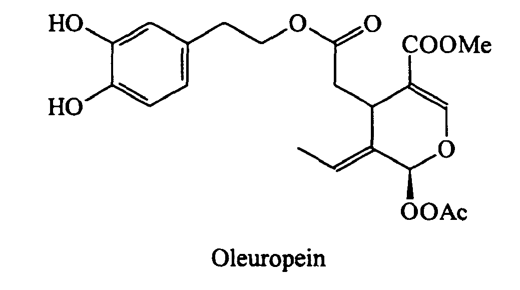

### • Glyzyrrhiza glabra

The licorice root contains glycyrrhizin, 50 times sweeter than sucrose, which encourages the production of hormones such as hydrocortisone. The extracts show an anti-inflammatory action and also stimulate the adrenal cortex after steroid therapy. Main component of the extracts of *Glyzyrrhiza glabra* is glycyrrhizinic acid:

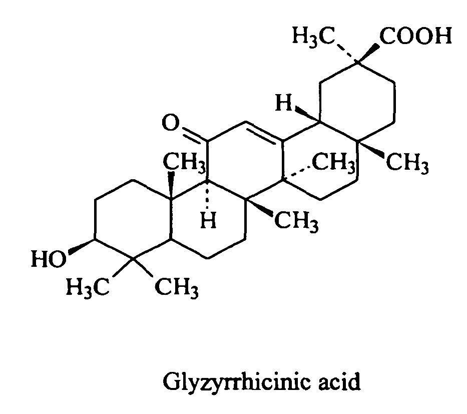

Beside the acid, also their salts, mainly the zinc salts, as well as their esters (e.g. with fatty alcohols or sterols) can be employed.

### • Vaccinium myrtillus

Extracts of blueberries (*Vaccinium myrtillus*) comprise a mixture of at least 15 different anthocyanosides, like the following.

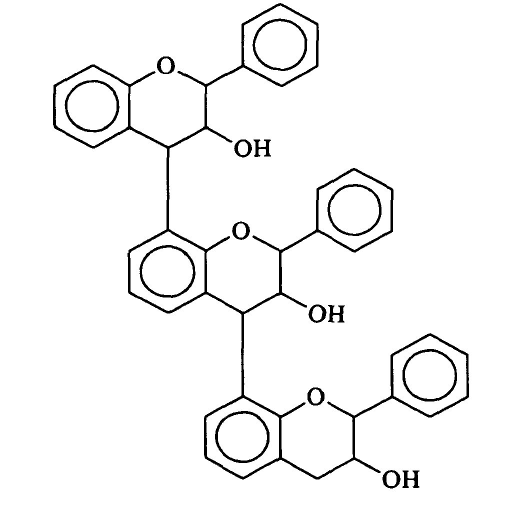

Usually, extracts of Vaccinium comprise 20 to 25% b.w. anthocyanosides, 5 to 10 % b.w. tannins and small amounts of various alkaloids like e.g. myrtin and epimyrtin, phenolic acids and glycosides of quercitrin, isoquercitrin and hyperosid.

### • Trifolium pratense

The main active principles of red clover (*Trifolium pratense*) are isoflavones, like e.g. daidzein, genestein, formononentin and biochanin as well as their glucosides like ononin or sissostrin:

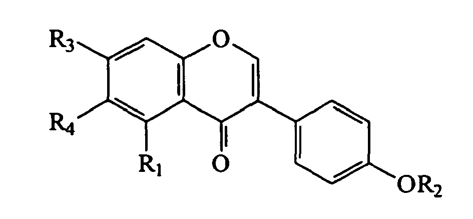

| *Isoflavonglucosides | R₁ | R₂ | R₃ | R₄ |
|---|---|---|---|---|
| Daizidin | H | H | Glucose | H |
| Genistin | H | H | Glucose | OH |
| Ononin | H | CH₃ | Glucose | H |
| Sissostrin | H | CH₃ | Glucose | OH |

### • Litchi sinensis

Extracts of pericarps from Litchi *(Litchi sinensis)* are well known for its high content of flavon derivatives like e.g. 2-phenyl-4H-1-benzopyrans, flavanen, flavan-3-ols (catechins, catechin oligomeren), flavan-3,4-diols (leucoanthocyaniden), flavons, flavonols and flavonons. The main component, however, represent condensed tannins, so-called procyanodols (OPC). These compounds comprise 2 to 8 monomers of the catechin or epicatechin-type, like e.g. procyanidins, proanthocynidins, procyanidoel, oligoprocyanidins, leucoanthocyanidins, leucodelphinins, leucocyanins and anthocyanogens. OPC, mainly the preferred proanthocyanidin A2 (OPC A2) behave like vitamin P, especially with respect to MMP inhibition.

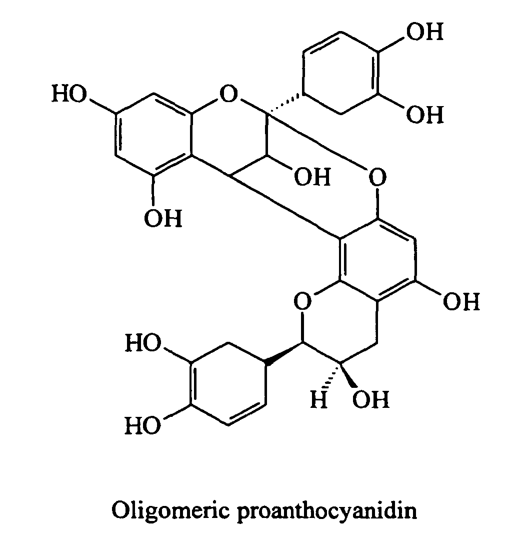

### • Vitis vinifera, Brassica oleracea, and Punica granatum

The main actives of grape vine (*Vitis vinifera*) are polyphenols of the OPC type. The main active principles of cauliflower *(Brassica oleracea)* are amino acids, especially methionin and cystein, and glucosinolates like e.g. glucoraphanin. The main active principles of grenadine (*Punica granatum*) are sugars, citric acid and delphinidin-1,2-glykoside or its aglykon

### • Petroselinium crispum

Main constituent of the fatty oil of parsil *(Petroselinium crispum)* is petroselinic acid. The extracts, however, show high contents of apiol (1-allyl-2,5-dimethoxy-3,4-(methylendioxy)benzol), and in addition of apiin, myristicin, pinen und selinen.

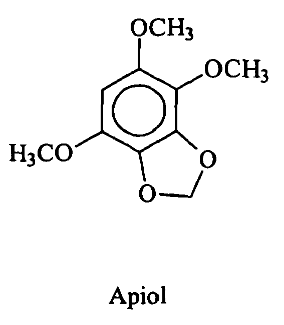

### • Centella asiatica

Main constituents of *Centella asiatica* are high condensed naphthenic acids, especially asiatica acid and madecassica acid and their glycosides.

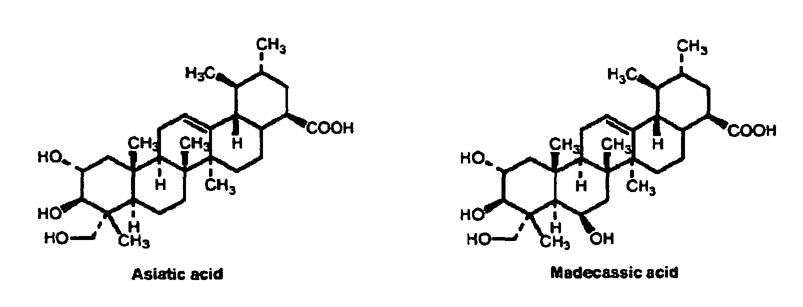

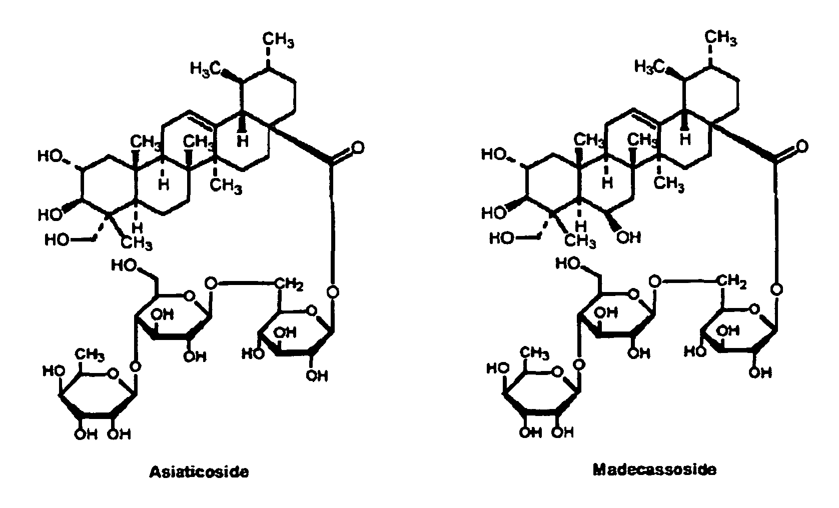

### • Passiflora incarnata

Extracts of passion flower (*Passiflora incarnata*) are rich in flavons of the apigenin and luteolin-type and their C-glycosides:

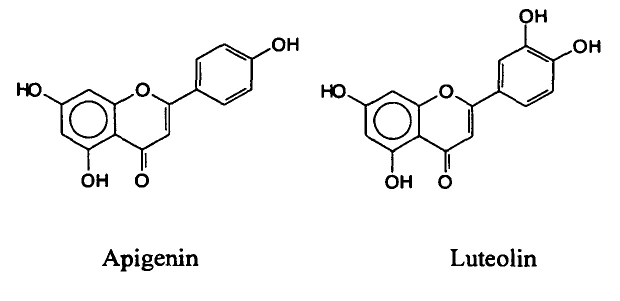

In addition they comprise 2"-B-D-glucosides, schaftosides and iso-schaftosides, isovitexin, isoorientin, vicenin-2, incenin-2, daponanin and trace elements like calcium, phosphor and iron.

### • Medicago sativa

Extracts of Alfalfa (*Medicago sativa*) are rich in isoflavons like e.g. daidzein, genestein, formononetin, biochanin A und tricin :

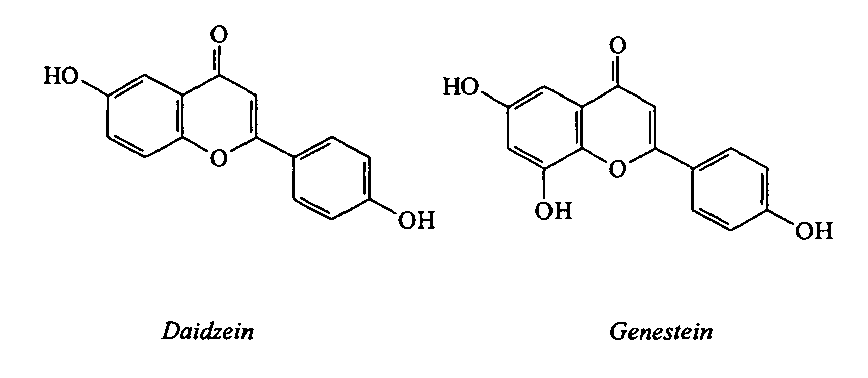

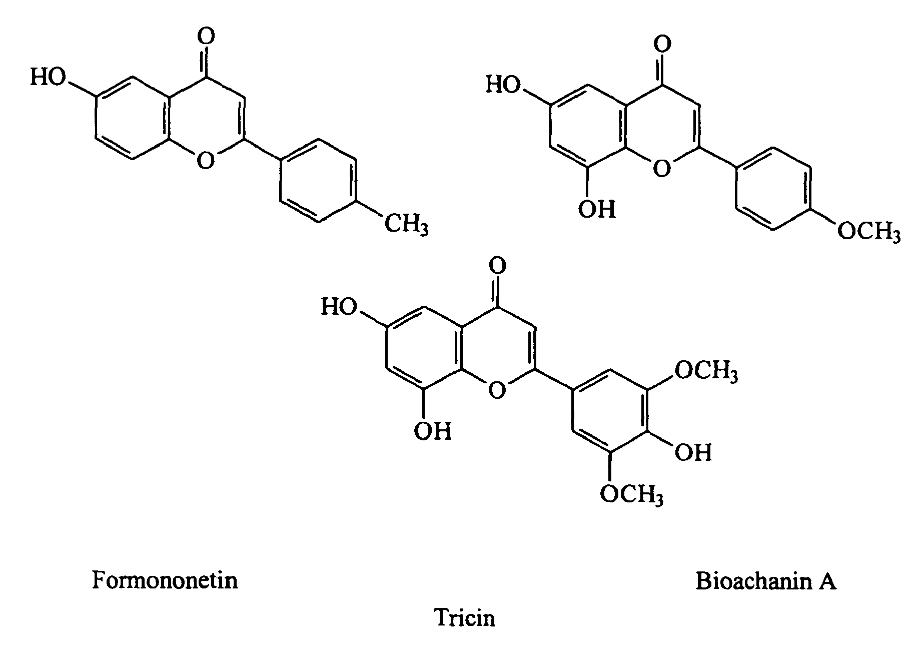

### • Valeriana officinalis

The main constituents of extracts of *Valeriana officinalis* are valeric acid, valerianone and borneol esters.

### • Castanea sativa

Main ingredients of horse chestnuts (*Castanea sativa*) are saponins and escin, which is a mixture of two glycosides, whose aglycons are derived from proteoescigenin, while the sugars represent either glucoronic acid of two molecules D-glucose. Said glycosides differ in the acyl groups in the C22-position.

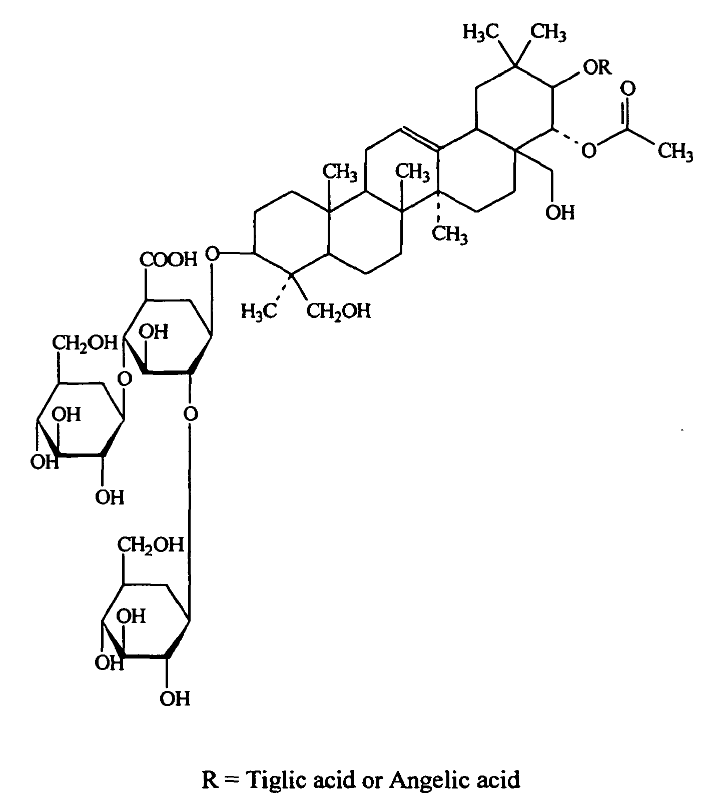

While α-escin represents an amorphous powder, which melts between 225 and 227 °C and is easily soluble in water, β-escin (which is also called flogencyl) forms flakes, which are practically water-insoluble, but can be dissolved in alcohol.

### • Salix alba

Main constituents of *Salix alba* are phenolic glycosides and especially salicylates like e.g. salicin, salicortin and tremulacin:

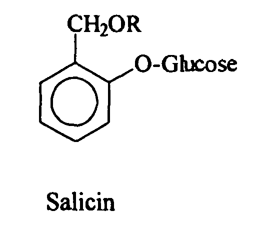

### • Harpagophytum procumbens

The main active principles of devil's craw *(Harpagophytum procumbens)* are iridoidglucosides, harpagosides, harpagides and procumbides.

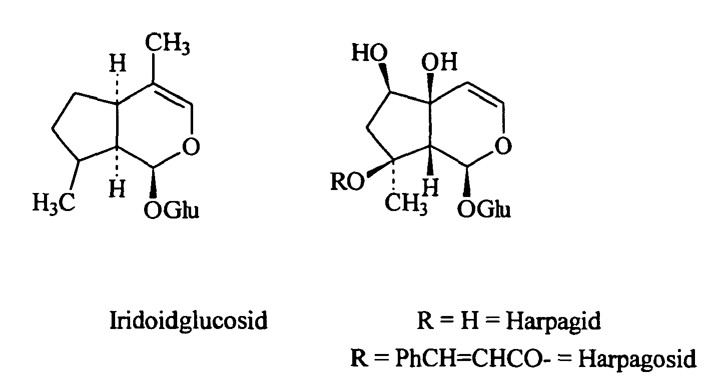

In addition one finds stachylose, free and glycosylated phytosterols (e.g. β-sitosterol), flavonoides (e.g. kaempferol, luteolin), phenolic acids und glycosidic phenylpropanoicacid esters (e.g. verbacosides, isoacteosides). Pigments

Although the selection of suitable pigments (component b2) is not a critical feature and is obviously linked to the colour of the capsule, the preferred pigments are selected from the group consisting of chlorophyll, titanium dioxide, zinc oxide, iron oxide, chromium oxides, chromium hydroxides, ultramarines, indogo pigments, manganese ammonium pyrophosphates, copper phtalocyanines and mica, since these compounds offer an additional benefit when the capsules are applied to skin.

### Vitamines

Vitamines (component b3) are usually chosen from the group consisting of carotinoids and ascorbic acid. Among the carotinoids usually carotines are understood. Carotins represent a group of unsaturated terpenes comprising 11 or 12 double bonds. Of special importance are the three isomeric α-, β- und γ-carotines which all show the same carbon chain skeleton having 9 conjugated double bonds, 8 methyl groups in the side chain (including ring structures) and a β-ionon moiety at the end of the molecule and originally have been considered to be a single component. In the following a couple of carotins are shown which come also into account, although this list must be regarded as illustrating, but not limiting:

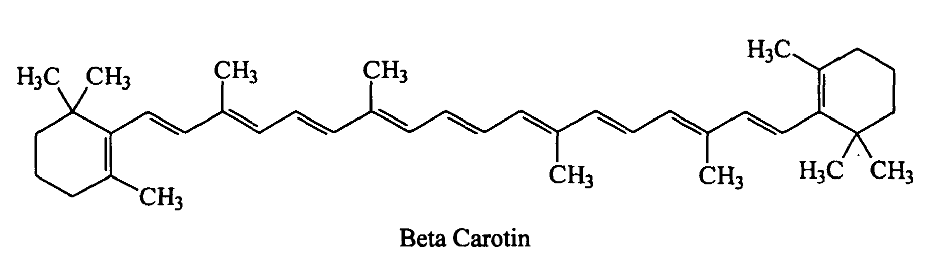

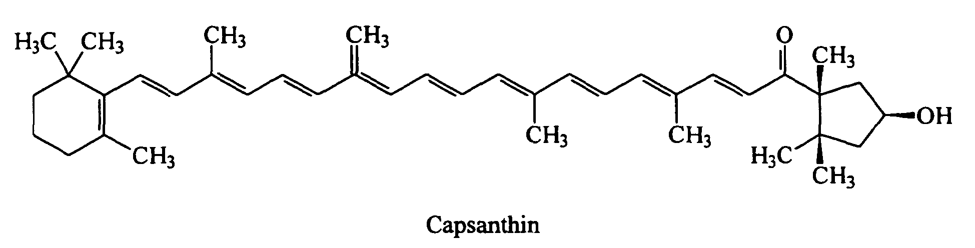

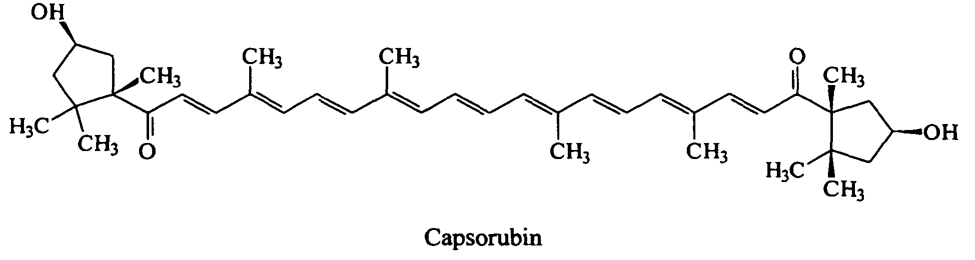

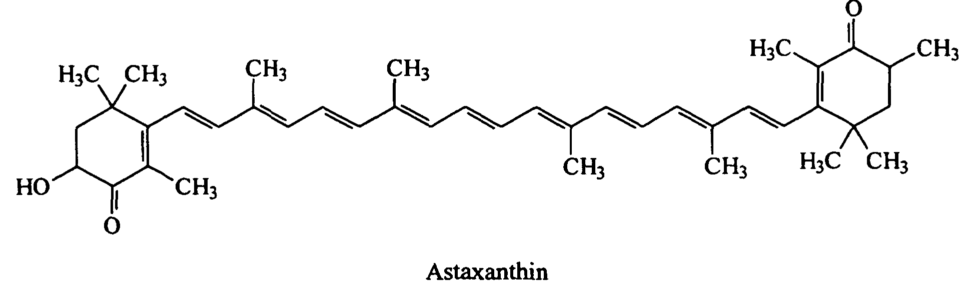

Beside the isomers already mentioned one can also take δ-, ε- und ζ-Carotin (lycopene) into consideration, although of course β-carotin (provitamine A) has the highest relevance due to the fact that it can be obtained from large resources. In the organism it is split into two molecules of retinal.

Derivatives of carotins comprising oxygen are usually called xanthophylls and are based on a skeleton of 8 isopren units (tetraterpenes). Typical examples are (3R,6'R)-β-ε-Carotin-3,3'-diol (Lutein), (3R,3'S,5'R)-3,3'-Dihydroxy-β,κ-carotin-6-on (Capsanthin), 9'-cis-6,6'-Diapocarotindiacid-6'-methylester (Bixin), (3S,3'S,5R,5'R)-3,3'-Dihydroxy-κ,κ-carotin-6,6'-dion (Capsorubin) and 3S,3'S)-3,3'-Dihydroxy-β,β'-carotin-4,4'-dion (Astaxanthin). Other carotinoids which can be useful for the present invention are for example 3,7-Dimethyl-9-(2,6,6-trimethyl-1-cyclohexeny)-2,4,6,8-nonatetraen-1-ol (Retinol, Vitamine A1) and 3,7-Dimethyl-9-(2,6,6-trimethyl-1-cyclohexenyl)-2,4,6,8-nonatetraenal (Retinal, Vitamine A1-Aldehyde).

### Active principle compositions

In another preferred embodiment of the invention, said active principles may be present in amounts from about 0.01 to about 25, preferably 0.1 to 15 and more preferably 1 to 10 % b.w. It has been found advantageous for the suggested purpose that the relation by weight between said exfoliating agents, preferably said bamboo extracts and said components (b) is from about 10 : 90 to about 99:1 preferably 25 : 75 to 75 : 25 and more preferably 40 : 60 to 60 : 40, while the relation by weight between the plant extracts (b1) on one hand and said pigments (b2) and/or vitamins (b3) on the other is from about 99 : 1 to about 50 : 50, and pref erably 95 : 5 to 75 : 25.

### Microcapsules

"Microcapsules" are understood to be spherical aggregates with a diameter of about 0.1 to about 5 mm which contain at least one solid or liquid core surrounded by at least one continuous membrane. More precisely, they are finely dispersed liquid or solid phases coated with film-forming polymers, in the production of which the polymers are deposited onto the material to be encapsulated after emulsification and coacervation or interfacial polymerization. In another process, liquid active principles are absorbed in a matrix ("microsponge") and, as microparticles, may be additionally coated with film-forming polymers. The microscopically small capsules, also known as nanocapsules, can be dried in the same way as powders. Besides single-core microcapsules, there are also multiple-core aggregates, also known as microspheres, which contain two or more cores distributed in the continuous membrane material. In addition, single-core or multiple-core microcapsules may be surrounded by an additional second, third etc. membrane. The membrane may consist of natural, semi-synthetic or synthetic materials. Natural membrane materials are, for example, gum arabic, agar agar, agarose, maltodextrins, alginic acid and salts thereof, for example sodium or calcium alginate, fats and fatty acids, cetyl alcohol, collagen, chitosan, lecithins, gelatin, albumin, shellac, polysaccharides, such as starch or dextran, polypeptides, protein hydrolyzates, sucrose and waxes. Semi-synthetic membrane materials are inter alia chemically modified celluloses, more particularly cellulose esters and ethers, for example cellulose acetate, ethyl cellulose, hydroxypropyl cellulose, hydroxypropyl methyl cellulose and carboxymethyl cellulose, and starch derivatives, more particularly starch ethers and esters. Synthetic membrane materials are, for example, polymers, such as polyacrylates, polyamides, polyvinyl alcohol or polyvinyl pyrrolidone.

Examples of known microcapsules are the following commercial products (the membrane material is shown in brackets) Hallcrest Microcapsules (gelatin, gum arabic), Coletica Thalaspheres (maritime collagen), Lipotec Millicapseln (alginic acid, agar agar), Induchem Unispheres (lactose, microcrystalline cellulose, hydroxypropylmethyl cellulose), Unicetin C30 (lactose, microcrystalline cellulose, hydroxypropylmethyl cellulose), Kobo Glycospheres (modified starch, fatty acid esters, phospholipids), Softspheres (modified agar agar) and Kuhs Probiol Nanospheres (phospholipids).

The active principles are released from the microcapsules by mechanical, thermal, chemical or enzymatic destruction of the membrane, normally during the use of the preparations containing the microcapsules. From the state of the art also a huge number of different processes for the encapsulation of active principles are known: WO 99/043426**;** WO 01/001928**;** WO 01/001929**;** WO 01/066240**;** WO 01/066241**;** WO 01/098578**;** WO 02/0178859**;** WO 02/0178868**;** WO 02/076607**;** WO 02/076606**;** WO 02/077359**;** WO 02/077360**;** WO 03/022419**;** WO 03/093571**;** WO 03/092664**;** WO 03/092880**;** WO 04/091555**;** WO 04/106621**;** EP 1064911 B1**;** EP 1064912 B1**;** EP 1077060 B1**;** EP 1101527 B1**;** EP 1223243 B1**;** EP 1243318 B1**;** EP 1243320 B1**;** EP 1243323 B1**;** EP 1243324 B1**;** EP 1254983 B1**;** EP 1121542 B1 all filed on behalf of Henkel KGaA, Primacare S.A. or Cognis Iberia, S.L..

Despite the fact that the state of the art a huge range of possibilities for the encapsulation of actives, methods according to which a shell is obtained by coazervation, precipitation or polycondensation of anionic and cationic monomers or polymers has been quite suitable for the formation of stable capsules. Particularly, a preferred process for the encapsulation of active principles according to the present invention is characterised in that it comprises the steps of
(a) preparing a matrix from gel formers, cationic polymers and active principles;
(b) optionally dispersing said matrix in an oil phase; and
(c) treating said dispersed matrix with aqueous solutions of anionic polymers and optionally removing the in phase in the process.

Of course, anionic and cationic polymers in steps (a) and (c) can be exchanged.

### • Gel formers

In the context of the invention, preferred gel formers are substances which are capable of forming gels in aqueous solution at temperatures above 40° C. Typical examples of such gel formers are heteropolysaccharides and proteins. Preferred thermogelling heteropolysaccharides are agaroses which may be present in the form of the agar agar obtainable from red algae, even together with up to 30% by weight of non-gel-forming agaropectins. The principal constituent of agaroses are linear polysaccharides of Galactose and 3,6-anhydro-L-galactose with alternate 1,3- and 1,4-glycosidic bonds. The heteropolysaccharides preferably have a molecular weight of 110,000 to 160,000 and are both odourless and tasteless. Suitable alternatives are pectins, xanthans (including xanthan gum) and mixtures thereof. Other preferred types are those which in 1% by weight aqueous solution still form gels that do not melt below 80° C. and solidify again above 40° C. Examples from the group of thermogelling proteins are the various gelatines.

### • Anionic polymers

Salts of alginic acid are preferred for this purpose. The alginic acid is a mixture of carboxyl-containing polysaccharides with the following idealized monomer unit:

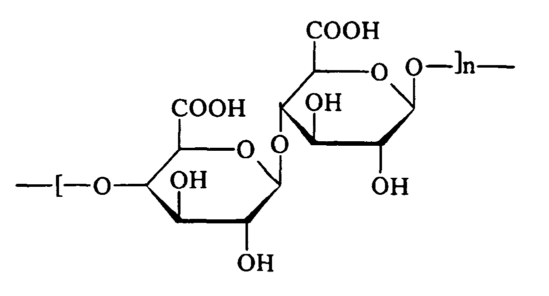

The average molecular weight of the alginic acid or the alginates is in the range from 150,000 to 250,000. Salts of alginic acid and complete and partial neutralization products thereof are understood In particular to be the alkali metal salts, preferably sodium alginate ("algin") and the ammonium and alkaline earth metal salts. Mixed alginates, for example sodium/magnesium or sodium/calcium alginates, are particularly preferred. In an alternative embodiment of the invention, however, anionic chitosan derivatives, for example the carboxylation and above all succinylation products are also suitable for this purpose. Other suitable anionic polymers are carboxymethylcelluloses, gellan gum and acrylates.

### • Cationic polymers

Chitosans are biopolymers which belong to the group of hydrocolloids. Chemically, they are partly de-acetylated chitins differing in their molecular weights which contain the following ― idealized - monomer unit:

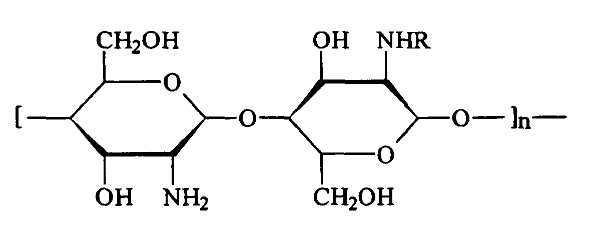

In contrast to most hydrocolloids, which are negatively charged at biological pH values, chitosans are cationic biopolymers under these conditions. The positively charged chitosans are capable of interacting with oppositely charged surfaces and are therefore used in cosmetic hair- care and body-care products and pharmaceutical preparations. Chitosans are produced from chitin, preferably from the shell residues of crustaceans which are available in large quantities as inexpensive raw materials. In a process described for the first time by Hackmann et al., the chitin is normally first de-proteinized by addition of bases, demineralized by addition of mineral acids and, finally, de-acetylated by addition of strong bases, the molecular weights being distributed over a broad spectrum. Preferred types are those which are disclosed in German patent applications DE 4442987 A1 and DE 19537001 A1 (Henkel) and which have an average molecular weight of 10,000 to 500,000 Dalton or 800,000 to 1,200,000 Dalton and/or a Brookfield viscosity (1 % by weight in glycolic acid) below 5,000 mPas, a degree of de-acetylation of 80 to 88 % and an ash content of less than 0.3% by weight. In the interests of better solubility in water, the chitosans are generally used in the form of their salts, preferably as glycolates. Other suitable cationic polymers are those known for use especially for cosmetics, preferably polyquternium or gelatin.

In a preferred embodiment of the invention a 1 to 10 and preferably 2 to 5% by weight aqueous solution of the gel former, preferably agar agar, is normally prepared and heated under reflux. A second aqueous solution containing the cationic polymer, preferably chitosan, in quantities of 0.1 to 2 and preferably 0.25 to 0.5% by weight and the active principle in quantities of 0.1 to 25 and preferably 0.25 to 10% by weight is added in the boiling heat, preferably at 80 to 100 ° C.; this mixture is called the matrix. Accordingly, the charging of the microcapsules with active principles may also comprise 0.1 to 25% by weight, based on the weight of the capsules. If desired, water-insoluble constituents, for example inorganic pigments, may also be added at this stage to adjust viscosity, generally in the form of aqueous or aqueous/alcoholic dispersions. In addition, to emulsify or disperse the active principles, it can be useful to add emulsifiers and/or solubilisers to the matrix. After its preparation from gel former, cationic polymer and active principle, the matrix optionally is very finely dispersed in an oil phase with intensive shearing in order to produce small particles in the subsequent encapsulation process. It has proved to be particularly advantageous in this regard to heat the matrix to temperatures in the range from 40 to 60° C while the oil phase is cooled to 10 to 20° C. The actual encapsulation, i.e. formation of the membrane by contacting the cationic polymer in the matrix with the anionic polymers, takes place in the third step. To this end, it is advisable to wash the matrix - dispersed in the oil phase - with an aqueous ca. 0.1 to 3 and preferably 0.25 to 0.5% by weight aqueous solution of the anionic polymer, preferably the alginate, at a temperature in the range from 40 to 100 and preferably 50 to 60° C. and, at the same time, to remove the oil phase if present. The resulting aqueous preparations generally have a microcapsule content of 1 to 10% by weight. In some cases, it can be of advantage for the solution of the polymers to contain other ingredients, for example emulsifiers or preservatives. After filtration, microcapsules with a mean diameter of preferably 1 to 3 mm are obtained. It is advisable to sieve the capsules to ensure a uniform size distribution. The microcapsules thus obtained may have any shape within production-related limits, but are preferably substantially spherical.

### Skin care compositions

Two other feature of the present invention are related to cosmetic compositions, more particularly skin care compositions comprising said new microcapsules and the use of said microcapsules for making cosmetic compositions, particularly skin care compositions.

The preparations according to the invention may contain surfactants, oil bodies, emulsifiers, superfatting agents, pearlising waxes, consistency factors, polymers, silicone compounds, waxes, stabilizers, primary and secondary sun protection agents, biogenic agents, film formers, swelling agents, hydrotropes, preservatives, solubilizers, perfume oils, dyes and the like as additional auxiliaries and additives.

### Surfactants

Other preferred auxiliaries and additives are anionic and/or amphoteric or zwitterionic surfactants. Typical examples of anionic surfactants are soaps, alkyl benzenesulfonates, alkanesulfonates, olefin sulfonates, alkylether sulfonates, glycerol ether sulfonates, methyl ester sulfonates, sulfofatty acids, alkyl sulfates, fatty alcohol ether sulfates, glycerol ether sulfates, fatty acid ether sulfates, hydroxy mixed ether sulfates, monoglyceride (ether) sulfates, fatty acid amide (ether) sulfates, mono- and dialkyl sulfosuccinates, mono- and dialkyl sulfosuccinamates, sulfotriglycerides, amide soaps, ether carboxylic acids and salts thereof, fatty acid isethionates, fatty acid sarcosinates, fatty acid taurides, N-acylamino acids such as, for example, acyl lactylates, acyl tartrates, acyl glutamates and acyl aspartates, alkyl oligoglucoside sulfates, protein fatty acid condensates (particularly wheat-based vegetable products) and alkyl (ether) phosphates. If the anionic surfactants contain polyglycol ether chains, they may have a conventional homolog distribution although they preferably have a narrow-range homolog distribution. Typical examples of amphoteric or zwitterionic surfactants are alkylbetaines, alkylamidobetaines, aminopropionates, aminoglycinates, imidazolinium betaines and sulfobetaines. The surfactants mentioned are all known compounds. Information on their structure and production can be found in relevant synoptic works, cf. for example J. Falbe (ed.), "Surfactants in Consumer Products", Springer Verlag, Berlin, 1987, pages 54 to 124 **or** J. Falbe (ed.), "Katalysatoren, Tenside und Mineralöladditive (Catalysts, Surfactants and Mineral Oil Additives)", Thieme Verlag, Stuttgart, 1978, pages 123-217**.** The percentage content of surfactants in the preparations may be from 0.1 to 10% by weight and is preferably from 0.5 to 5% by weight, based on the preparation.

### Oil bodies

Suitable oil bodies, which form constituents of the O/W emulsions, are, for example, Guerbet alcohols based on fatty alcohols having 6 to 18, preferably 8 to 10, carbon atoms, esters of linear C₆-C₂₂-fatty acids with linear or branched C₆-C₂₂-fatty alcohols or esters of branched C₆-C ₁₃-carboxylic acids with linear or branched C₆-C ₂₂-fatty alcohols, such as, for example, myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate. Also suitable are esters of linear C₆-C₂₂-fatty acids with branched alcohols, in particular 2-ethylhexanol, esters of C₁₈-C₃₈- alkylhydroxy carboxylic acids with linear or branched C₆-C ₂₂-fatty alcohols, in particular Dioctyl Malate, esters of linear and/or branched fatty acids with polyhydric alcohols (such as, for example, propylene glycol, dimerdiol or trimertriol) and/or Guerbet alcohols, triglycerides based on C₆ -C₁₀-fatty acids, liquid mono-/di-/triglyceride mixtures based on C₆-C₁₈-fatty acids, esters of C₆- C₂₂-fatty alcohols and/or Guerbet alcohols with aromatic carboxylic acids, in particular benzoic acid, esters of C₂- C₁₂-dicarboxylic acids with linear or branched alcohols having 1 to 22 carbon atoms or polyols having 2 to 10 carbon atoms and 2 to 6 hydroxyl groups, vegetable oils, branched primary alcohols, substituted cyclohexanes, linear and branched C₆-C₂₂-fatty alcohol carbonates, such as, for example, Dicaprylyl Carbonate (Cetiol® CC), Guerbet carbonates, based on fatty alcohols having 6 to 18, preferably 8 to 10, carbon atoms, esters of benzoic acid with linear and/or branched C₆-C₂₂-alcohols (e.g. Finsolv® TN), linear or branched, symmetrical or asymmetrical dialkyl ethers having 6 to 22 carbon atoms per alkyl group, such as, for example, dicaprylyl ether (Cetiol® OE), ring-opening products of epoxidized fatty acid esters with polyols, silicone oils (cyclomethicones, silicone methicone grades, etc.) and/or aliphatic or naphthenic hydrocarbons, such as, for example, squalane, squalene or dialkylcyclohexanes. Emulsifiers

Other surfactants may also be added to the preparations as emulsifiers, including for example:
- products of the addition of 2 to 30 mol ethylene oxide and/or 0 to 5 mol propylene oxide onto linear C₈₋₂₂ fatty alcohols, onto C₁₂₋₂₂ fatty acids and onto alkyl phenols containing 8 to 15 carbon atoms in the alkyl group;
- C_{12/18} fatty acid monoesters and diesters of addition products of 1 to 30 mol ethylene oxide onto glycerol;
- glycerol mono- and diesters and sorbitan mono- and diesters of saturated and unsaturated fatty acids containing 6 to 22 carbon atoms and ethylene oxide addition products thereof;
- addition products of 15 to 60 mol ethylene oxide onto castor oil and/or hydrogenated castor oil;
- polyol esters and, in particular, polyglycerol esters such as, for example, polyglycerol polyricinoleate, polyglycerol poly-12-hydroxystearate or polyglycerol dimerate isostearate. Mixtures of compounds from several of these classes are also suitable;
- addition products of 2 to 15 mol ethylene oxide onto castor oil and/or hydrogenated castor oil;
- partial esters based on linear, branched, unsaturated or saturated C_{6/22} fatty acids, ricinoleic acid and 12-hydroxystearic acid and glycerol, polyglycerol, pentaerythritol, - dipentaerythritol, sugar alcohols (for example sorbitol), alkyl glucosides (for example methyl glucoside, butyl glucoside, lauryl glucoside) and polyglucosides (for example cellulose);
- mono-, di and trialkyl phosphates and mono-, di- and/or tri-PEG-alkyl phosphates and salts thereof;
- wool wax alcohols;
- polysiloxane/polyalkyl polyether copolymers and corresponding derivatives;
- mixed esters of pentaerythritol, fatty acids, citric acid and fatty alcohol and/or mixed esters of C₆₋₂₂ fatty acids, methyl glucose and polyols, preferably glycerol or polyglycerol,
- polyalkylene glycols and
- glycerol carbonate.

The addition products of ethylene oxide and/or propylene oxide onto fatty alcohols, fatty acids, alkylphenols, glycerol mono- and diesters and sorbitan mono- and diesters of fatty acids or onto castor oil are known commercially available products. They are homologue mixtures of which the average degree of alkoxylation corresponds to the ratio between the quantities of ethylene oxide and/or propylene oxide and substrate with which the addition reaction is carried out. C_{12/18} fatty acid monoesters and diesters of addition products of ethylene oxide onto glycerol are known as lipid layer enhancers for cosmetic formulations. The preferred emulsifiers are described in more detail as follows:

### • Partial glycerides

Typical examples of suitable partial glycerides are hydroxystearic acid monoglyceride, hydroxystearic acid diglyceride, isostearic acid monoglyceride, isostearic acid diglyceride, oleic acid monoglyceride, oleic acid diglyceride, ricinoleic acid monoglyceride, ricinoleic acid diglyceride, linoleic acid monoglyceride, linoleic acid diglyceride, linolenic acid monoglyceride, linolenic acid diglyceride, erucic acid monoglyceride, erucic acid diglyceride, tartaric acid monoglyceride, tartaric acid diglyceride, citric acid monoglyceride, citric acid diglyceride, malic acid monoglyceride, malic acid diglyceride and technical mixtures thereof which may still contain small quantities of triglyceride from the production process. Addition products of 1 to 30 and preferably 5 to 10 mol ethylene oxide onto the partial glycerides mentioned are also suitable.

### • Sorbitan esters

Suitable sorbitan esters are sorbitan monoisostearate, sorbitan sesquiisostearate, sorbitan diisostearate, sorbitan triisostearate, sorbitan monooleate, sorbitan sesquioleate, sorbitan dioleate, sorbitan trioleate, sorbitan monoerucate, sorbitan sesquierucate, sorbitan dierucate, sorbitan trierucate, sorbitan monoricinoleate, sorbitan sesquiricinoleate, sorbitan diricinoleate, sorbitan triricinoleate, sorbitan monohydroxystearate, sorbitan sesquihydroxystearate, sorbitan dihydroxystearate, sorbitan trihydroxystearate, sorbitan monotartrate, sorbitan sesquitartrate, sorbitan ditartrate, sorbitan tritartrate, sorbitan monocitrate, sorbitan sesquicitrate, sorbitan dicitrate, sorbitan tricitrate, sorbitan monomaleate, sorbitan sesquimaleate, sorbitan dimaleate, sorbitan trimaleate and technical mixtures thereof. Addition products of 1 to 30 and preferably 5 to 10 mol ethylene oxide onto the sorbitan esters mentioned are also suitable.

### • Polyglycerol esters

Typical examples of suitable polyglycerol esters are Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) and Polyglyceryl Polyricinoleate (Admul® WOL 1403), Polyglyceryl Dimerate Isostearate and mixtures thereof. Examples of other suitable polyolesters are the mono-, di- and triesters of trimethylol propane or pentaerythritol with lauric acid, cocofatty acid, tallow fatty acid, palmitic acid, stearic acid, oleic acid, behenic acid and the like optionally reacted with 1 to 30 mol ethylene oxide.

Typical anionic emulsifiers are aliphatic C₁₂₋₂₂ fatty acids, such as palmitic acid, stearic acid or behenic acid for example, and C₁₂₋₂₂ dicarboxylic acids, such as azelaic acid or sebacic acid for example.

Other suitable emulsifiers are zwitterionic surfactants. Zwitterionic surfactants are surface-active compounds which contain at least one quaternary ammonium group and at least one carboxylate and one sulfonate group in the molecule. Particularly suitable zwitterionic surfactants are the so-called betaines, such as the N-alkyl-N,N-dimethyl ammonium glycinates, for example cocoalkyl dimethyl ammonium glycinate, N-acylaminopropyl-N,N-dimethyl ammonium glycinates, for example cocoacylaminopropyl dimethyl ammonium glycinate, and 2-alkyl-3-carboxymethyl-3-hydroxyethyl imidazolines containing 8 to 18 carbon atoms in the alkyl or acyl group and cocoacylaminoethyl hydroxyethyl carboxymethyl glycinate. The fatty acid amide derivative known under the CTFA name of *Cocamidopropyl Betaine* is particularly preferred. Ampholytic surfactants are also suitable emulsifiers. Ampholytic surfactants are surface-active compounds which, in addition to a C_{8/18} alkyl or acyl group, contain at least one free amino group and at least one ―COOH- or -SO₃H- group in the molecule and which are capable of forming inner salts. Examples of suitable ampholytic surfactants are N-alkyl glycines, N-alkyl propionic acids, N-alkylaminobutyric acids, N-alkyliminodipropionic acids, N-hydroxyethyl-N-alkylamidopropyl glycines, N-alkyl taurines, N-alkyl sarcosines, 2-alkylaminopropionic acids and alkylaminoacetic acids containing around 8 to 18 carbon atoms in the alkyl group. Particularly preferred ampholytic surfactants are N-cocoalkylaminopropionate, cocoacylaminoethyl aminopropionate and C_{12/18} acyl sarcosine.

### Superfatting agents

Superfatting agents may be selected from such substances as, for example, lanolin and lecithin and also polyethoxylated or acylated lanolin and lecithin derivatives, polyol fatty acid esters, monoglycerides and fatty acid alkanolamides, the fatty acid alkanolamides also serving as foam stabilizers.

### Consistency factors

The consistency factors mainly used are fatty alcohols or hydroxyfatty alcohols containing 12 to 22 and preferably 16 to 18 carbon atoms and also partial glycerides, fatty acids or hydroxyfatty acids. A combination of these substances with alkyl oligoglucosides and/or fatty acid N-methyl glucamides of the same chain length and/or polyglycerol poly-12-hydroxystearates is preferably used.

### Thickening agents

Suitable thickeners are polymeric thickeners, such as Aerosil® types (hydrophilic silicas), polysaccharides, more especially xanthan gum, guar-guar, agar-agar, alginates and tyloses, carboxymethyl cellulose and hydroxyethyl cellulose, also relatively high molecular weight polyethylene glycol monoesters and diesters of fatty acids, polyacrylates (for example Carbopols® [Goodrich] or Synthalens® [Sigma]), polyacrylamides, polyvinyl alcohol and polyvinyl pyrrolidone, surfactants such as, for example, ethoxylated fatty acid glycerides, esters of fatty acids with polyols, for example pentaerythritol or trimethylol propane, narrow-range fatty alcohol ethoxylates and electrolytes, such as sodium chloride and ammonium chloride.

### Polymers

Suitable cationic polymers are, for example, cationic cellulose derivatives such as, for example, the quaternized hydroxyethyl cellulose obtainable from Amerchol under the name of Polymer JR 400®, cationic starch, copolymers of diallyl ammonium salts and acrylamides, quaternized vinyl pyrrolidone/vinyl imidazole polymers such as, for example, Luviquat® (BASF), condensation products of polyglycols and amines, quaternized collagen polypeptides such as, for example, Lauryldimonium Hydroxypropyl Hydrolyzed Collagen (Lamequat® L, Grünau), quaternized wheat polypeptides, polyethyleneimine, cationic silicone polymers such as, for example, amodimethicone, copolymers of adipic acid and dimethylaminohy-droxypropyl diethylenetriamine (Cartaretine®, Sandoz), copolymers of acrylic acid with dimethyl diallyl ammonium chloride (Merquat® 550, Chemviron), polyaminopolyamides and crosslinked water-soluble polymers thereof, cationic chitin derivatives such as, for example, quaternized chitosan, optionally in microcrystalline distribution, condensation products of dihaloalkyls, for example dibromobutane, with bis-dialkylamines, for example bis-dimethylamino-1,3-propane, cationic guar gum such as, for example, Jaguar®CBS, Jaguar®C-17, Jaguar®C-16 of Celanese, quaternized ammonium salt polymers such as, for example, Mirapol® A-15, Mirapol® AD-1, Mirapol® AZ-1 of Miranol.

Suitable anionic, zwitterionic, amphoteric and nonionic polymers are, for example, vinyl acetate/crotonic acid copolymers, vinyl pyrrolidone/vinyl acrylate copolymers, vinyl acetate/butyl maleate/isobornyl acrylate copolymers, methyl vinylether/maleic anhydride copolymers and esters thereof, uncrosslinked and polyol-crosslinked polyacrylic acids, acrylamidopropyl trimethylammonium chloride/acrylate copolymers, octylacrylamide/methyl methacrylate/tert.-butylaminoethyl methacrylate/2-hydroxypropyl methacrylate copolymers, polyvinyl pyrrolidone, vinyl pyrrolidone/vinyl acetate copolymers, vinyl pyrrolidone/dimethylaminoethyl methacrylate/vinyl caprolactam terpolymers and optionally derivatized cellulose ethers and silicones.

### Pearlising waxes

Suitable pearlising waxes are, for example, alkylene glycol esters, especially ethylene glycol distearate; fatty acid alkanolamides, especially cocofatty acid diethanolamide; partial glycerides, especially stearic acid monoglyceride; esters of polybasic, optionally hydroxy-substituted carboxylic acids with fatty alcohols containing 6 to 22 carbon atoms, especially long-chain esters of tartaric acid; fatty compounds, such as for example fatty alcohols, fatty ketones, fatty aldehydes, fatty ethers and fatty carbonates which contain in all at least 24 carbon atoms, especially laurone and distearylether; fatty acids, such as stearic acid, hydroxystearic acid or behenic acid, ring opening products of olefin epoxides containing 12 to 22 carbon atoms with fatty alcohols containing 12 to 22 carbon atoms and/or polyols containing 2 to 15 carbon atoms and 2 to 10 hydroxyl groups and mixtures thereof.

### Silicones

Suitable silicone compounds are, for example, dimethyl polysiloxanes, methylphenyl polysiloxanes, cyclic silicones and amino-, fatty acid-, alcohol-, polyether-, epoxy-, fluorine-, glycoside- and/or alkyl-modified silicone compounds which may be both liquid and resin-like at room temperature. Other suitable silicone compounds are simethicones which are mixtures of dimethicones with an average chain length of 200 to 300 dimethylsiloxane units and hydrogenated silicates. A detailed overview of suitable volatile silicones can be found in Todd et al. in Cosm. Toil. 91, 27 (1976**).**

### Waxes

Besides natural oils used, waxes may also be present in the preparations, more especially natural waxes such as, for example, candelilla wax, carnauba wax, Japan wax, espartograss wax, cork wax, guaruma wax, rice oil wax, sugar cane wax, ouricury wax, montan wax, beeswax, shellac wax, spermaceti, lanolin (wool wax), uropygial fat, ceresine, ozocerite (earth wax), petrolatum, paraffin waxes and microwaxes; chemically modified waxes (hard waxes) such as, for example, montan ester waxes, sasol waxes, hydrogenated jojoba waxes and synthetic waxes such as, for example, polyalkylene waxes and polyethylene glycol waxes.

### Stabilizers

Metal salts of fatty acids such as, for example, magnesium, aluminium and/or zinc stearate or ricinoleate may be used as stabilizers.

### Primary sun protection factors

Primary sun protection factors in the context of the invention are, for example, organic substances (light filters) which are liquid or crystalline at room temperature and which are capable of absorbing ultraviolet radiation and of releasing the energy absorbed in the form of longer-wave radiation, for example heat. UV-B filters can be oil-soluble or water-soluble. The following are examples of oil-soluble substances:
- 3-benzylidene camphor or 3-benzylidene norcamphor and derivatives thereof, for example 3-(4-methylbenzylidene)-camphor;
- 4-aminobenzoic acid derivatives, preferably 4-(dimethylamino)benzoic acid-2-ethylhexyl ester, 4-(dimethylamino)-benzoic acid-2-octyl ester and 4-(dimethylamino)benzoic acid amyl ester;
- esters of cinnamic acid, preferably 4-methoxycinnamic acid-2-ethylhexyl ester, 4-methoxycinnamic acid propyl ester, 4-methoxycinnamic acid isoamyl ester, 2-cyano-3,3-phenylcinnamic acid-2-ethylhexyl ester (Octocrylene);
- esters of salicylic acid, preferably salicylic acid-2-ethylhexyl ester, salicylic acid-4-isopropylbenzyl ester, salicylic acid homomenthyl ester;
- derivatives of benzophenone, preferably 2-hydroxy-4-methoxybenzophenone, 2-hydroxy-4-methoxy-4'-methylbenzophenone, 2,2'-dihydroxy-4-methoxybenzophenone;
- esters of benzalmalonic acid, preferably 4-methoxybenzalmalonic acid di-2-ethylhexyl ester;
- triazine derivatives such as, for example, 2,4,6-trianilino-(p-carbo-2'-ethyl-1'-hexyloxy)-1,3,5-triazine and Octyl Triazone or Dioctyl Butamido Triazone (Uvasorb® HEB);
- propane-1,3-diones such as, for example, 1-(4-tert.butylphenyl)-3-(4'-methoxyphenyl)-propane-1,3-dione;
- ketotricyclo(5.2.1.0)decane derivatives.
   Suitable water-soluble substances are
- 2-phenylbenzimidazole-5-sulfonic acid and alkali metal, alkaline earth metal, ammonium, alkylammonium, alkanolammonium and glucammonium salts thereof;
- sulfonic acid derivatives of benzophenones, preferably 2-hydroxy-4-methoxybenzophenone-5-sulfonic acid and salts thereof;
- sulfonic acid derivatives of 3-benzylidene camphor such as, for example, 4-(2-oxo-3-bornylidenemethyl)-benzene sulfonic acid and 2-methyl-5-(2-oxo-3-bomylidene)-sulfonic acid and salts thereof.

Typical UV-A filters are, in particular, derivatives of benzoyl methane such as, for example, 1-(4'-tert.butylphenyl)-3-(4'-methoxyphenyl)-propane-1,3-dione, 4-tert.butyl-4'-methoxydibenzoyl methane (Parsol® 1789) or 1-phenyl-3-(4'-isopropylphenyl)-propane-1,3-dione and the enamine compounds (BASF). The UV-A and UV-B filters may of course also be used in the form of mixtures. Particularly favourable combinations consist of the derivatives of benzoyl methane, for example 4-tert.butyl-4'-methoxydibenzoyl methane (Parsol® 1789) and 2-cyano-3,3-phenylcinnamic acid-2-ethylhexyl ester (Octocrylene®), in combination with esters of cinnamic acid, preferably 4-methoxycinnamic acid-2-ethylhexyl ester and/or 4-methoxycinnamic acid propyl ester and/or 4-methoxycinnamic acid isoamyl ester. Combinations such as these are advantageously combined with water-soluble filters such as, for example, 2-phenylbenzimidazole-5-sulfonic acid and alkali metal, alkaline earth metal, ammonium, alkylammonium, alkanolammonium and glucammonium salts thereof.

### Secondary sun protection factors

Besides the groups of primary sun protection factors mentioned above, secondary sun protection factors of the antioxidant type may also be used. Secondary sun protection factors of the antioxidant type interrupt the photochemical reaction chain which is initiated when UV rays penetrate into the skin. Typical examples are amino acids (for example glycine, histidine, tyrosine, tryptophane) and derivatives thereof, imidazoles (for example urocanic acid) and derivatives thereof, peptides, such as D,L-carnosine, D-carnosine, L-carnosine and derivatives thereof (for example anserine), carotinoids, carotenes (for example alpha-carotene, betacarotene, lycopene) and derivatives thereof, chlorogenic acid and derivatives thereof, liponic acid and derivatives thereof (for example dihydroliponic acid), aurothioglucose, propylthiouracil and other thiols (for example thioredoxine, glutathione, cysteine, cystine, cystamine and glycosyl, N-acetyl, methyl, ethyl, propyl, amyl, butyl and lauryl, palmitoyl, oleyl, alphalinoleyl, cholesteryl and glyceryl esters thereof) and their salts, dilaurylthiodipropionate, distearylthiodipropionate, thiodipropionic acid and derivatives thereof (esters, ethers, peptides, lipids, nucleotides, nucleosides and salts) and sulfoximine compounds (for example butionine sulfoximines, homocysteine sulfoximine, butionine sulfones, penta-, hexa- and hepta-thionine sulfoximine) in very small compatible dosages, also (metal) chelators (for example alphahydroxyfatty acids, palmitic acid, phytic acid, lactoferrine), alpha-hydroxy acids (for example citric acid, lactic acid, malic acid), humic acid, bile acid, bile extracts, bilirubin, biliverdin, EDTA, EGTA and derivatives thereof, unsaturated fatty acids and derivatives thereof (for example linoleic acid, oleic acid), folic acid and derivatives thereof, ubiquinone and ubiquinol and derivatives thereof, vitamin C and derivatives thereof (for example ascorbyl palmitate, Mg ascorbyl phosphate, ascorbyl acetate), tocopherols and derivatives (for example vitamin E acetate), vitamin A and derivatives (vitamin A palmitate) and coniferyl benzoate of benzoin resin, rutinic acid and derivatives thereof, glycosyl rutin, ferulic acid, furfurylidene glucitol, carnosine, butyl hydroxytoluene, butyl hydroxyanisole, nordihydroguaiac resin acid, nordihydroguaiaretic acid, trihydroxybutyrophenone, uric acid and derivatives thereof, mannose and derivatives thereof, superoxide dismutase, zinc and derivatives thereof (for example ZnO, ZnSO₄), selenium and derivatives thereof (for example selenium methionine), stilbenes and derivatives thereof (for example stilbene oxide, trans-stilbene oxide) and derivatives of these active substances suitable for the purposes of the invention (salts, esters, ethers, sugars, nucleotides, nucleosides, peptides and lipids).

### Biogenic agents

In the context of the invention, biogenic agents are, for example, tocopherol, tocopherol acetate, tocopherol palmitate, ascorbic acid, (deoxy)ribonucleic acid and fragmentation products thereof, β-glucans, retinol, bisabolol, allantoin, phytantriol, panthenol, AHA acids, amino acids, ceramides, pseudoceramides, essential oils, plant extracts, for example prune extract, bambara nut extract, and vitamin complexes.

### Anti-microbial agents

Suitable anti-microbial agents are, in principle, all substances effective against Gram-positive bacteria, such as, for example, 4- hydroxybenzoic acid and its salts and esters, N-(4-chlorophenyl)-N'-(3,4- dichlorophenyl)urea, 2,4,4'-trichloro-2'-hydroxy-diphenyl ether (triclosan), 4-chloro-3,5-dimethyl-phenol, 2,2'-methylenebis(6-bromo-4- chlorophenol), 3-methyl-4-(1-methylethyl)phenol, 2-benzyl-4-chloro-phenol, 3-(4-chlorophenoxy)-1,2-propanediol, 3-iodo-2-propynyl butylcarbamate, chlorhexidine, 3,4,4'-trichlorocarbanilide (TTC), antibacterial fragrances, thymol, thyme oil, eugenol, oil of cloves, menthol, mint oil, farnesol, phenoxyethanol, glycerol monocaprate, glycerol monocaprylate, glycerol monolaurate (GML), diglycerol monocaprate (DMC), salicylic acid N-alkylamides, such as, for example, n-octylsalicylamide or n- decylsalicylamide.

### Film formers

Standard film formers are, for example, chitosan, microcrystalline chitosan, quaternized chitosan, polyvinyl pyrrolidone, vinyl pyrrolidone/vinyl acetate copolymers, polymers of the acrylic acid series, quaternary cellulose derivatives, collagen, hyaluronic acid and salts thereof and similar compounds.

### Hydrotropes

In addition, hydrotropes, for example ethanol, isopropyl alcohol or polyols, may be used to improve flow behaviour. Suitable polyols preferably contain 2 to 15 carbon atoms and at least two hydroxyl groups. The polyols may contain other functional groups, more especially amino groups, or may be modified with nitrogen. Typical examples are
- glycerol;
- alkylene glycols such as, for example, ethylene glycol, diethylene glycol, propylene glycol, butylene glycol, hexylene glycol and polyethylene glycols with an average molecular weight of 100 to 1000 Dalton;
- technical oligoglycerol mixtures with a degree of self-condensation of 1.5 to 10, such as for example technical diglycerol mixtures with a diglycerol content of 40 to 50% by weight;
- methylol compounds such as, in particular, trimethylol ethane, trimethylol propane, trimethylol butane, pentaerythritol and dipentaerythritol;
- lower alkyl glucosides, particularly those containing 1 to 8 carbon atoms in the alkyl group, for example methyl and butyl glucoside;
- sugar alcohols containing 5 to 12 carbon atoms, for example sorbitol or mannitol,
- sugars containing 5 to 12 carbon atoms, for example glucose or sucrose;
- amino sugars, for example glucamine;
- dialcoholamines, such as diethanolamine or 2-aminopropane-1,3-diol.

### Preservatives

Suitable preservatives are, for example, phenoxyethanol, formaldehyde solution, parabens, pentanediol or sorbic acid and the other classes of compounds listed in Appendix 6, Parts A and B of the Kosmetikverordnung ("Cosmetics Directive").

### Perfume oils

Suitable perfume oils are mixtures of natural and synthetic perfumes. Natural perfumes include the extracts of blossoms (lily, lavender, rose, jasmine, neroli, ylang-ylang), stems and leaves (geranium, patchouli, petitgrain), fruits (anise, coriander, caraway, juniper), fruit peel (bergamot, lemon, orange), roots (nutmeg, angelica, celery, cardamom, costus, iris, calmus), woods (pinewood, sandalwood, guaiac wood, cedarwood, rosewood), herbs and grasses (tarragon, lemon grass, sage, thyme), needles and branches (spruce, fir, pine, dwarf pine), resins and balsams (galbanum, elemi, benzoin, myrrh, olibanum, opoponax). Animal raw materials, for example civet and beaver, may also be used. Typical synthetic perfume compounds are products of the ester, ether, aldehyde, ketone, alcohol and hydrocarbon type. Examples of perfume compounds of the ester type are benzyl acetate, phenoxyethyl isobutyrate, p-tert.butyl cyclohexylacetate, linalyl acetate, dimethyl benzyl carbinyl acetate, phenyl ethyl acetate, linalyl benzoate, benzyl formate, ethylmethyl phenyl glycinate, allyl cyclohexyl propionate, styrallyl propionate and benzyl salicylate. Ethers include, for example, benzyl ethyl ether while aldehydes include, for example, the linear alkanals containing 8 to 18 carbon atoms, citral, citronellal, citronellyloxyacetaldehyde, cyclamen aldehyde, hydroxycitronellal, lilial and bourgeonal. Examples of suitable ketones are the ionones, □-isomethylionone and methyl cedryl ketone. Suitable alcohols are anethol, citronellol, eugenol, isoeugenol, geraniol, linalool, phenylethyl alcohol and terpineol. The hydrocarbons mainly include the terpenes and balsams. However, it is preferred to use mixtures of different perfume compounds which, together, produce an agreeable perfume. Other suitable perfume oils are essential oils of relatively low volatility which are mostly used as aroma components. Examples are sage oil, camomile oil, clove oil, melissa oil, mint oil, cinnamon leaf oil, lime-blossom oil, juniper berry oil, vetiver oil, olibanum oil, galbanum oil, ladanum oil and lavendin oil. The following are preferably used either individually or in the form of mixtures: bergamot oil, dihydromyrcenol, lilial, lyral, citronellol, phenylethyl alcohol, hexylcinnamaldehyde, geraniol, benzyl acetone, cyclamen aldehyde, linalool, Boisambrene Forte, Ambroxan, indole, hedione, sandelice, citrus oil, mandarin oil, orange oil, allylamyl glycolate, cyclovertal, lavendin oil, clary oil, damascone, geranium oil bourbon, cyclohexyl salicylate, Vertofix Coeur, Iso-E-Super, Fixolide NP, evemyl, iraldein gamma, phenylacetic acid, geranyl acetate, benzyl acetate, rose oxide, romillat, irotyl and floramat.

### Dyes

Suitable dyes are any of the substances suitable and approved for cosmetic purposes as listed, for example, in the publication **"**Kosmetische Färbemittel" of the Farbstoffkommission der Deutschen Forschungsgemeinschaft, Verlag Chemie, Weinheim, 1984, pages 81 to 106**.** Examples include cochineal red A (C.I. 16255), patent blue V (C.I. 42051), indigotin (C.I. 73015), chlorophyllin (C.I. 75810), quinoline yellow (C.I. 47005), titanium dioxide (C.I. 77891), indanthrene blue RS (C.I. 69800) and madder lake (C.I. 58000). Luminol may also be present as a luminescent dye. These dyes are normally used in concentrations of 0.001 to 0.1 % by weight, based on the mixture as a whole.

The total percentage content of auxiliaries and additives may be from 1 to 50% by weight and is preferably from 5 to 40% by weight, based on the particular composition. The compositions may be produced by standard hot or cold processes.

### Examples

### Example 1

In a 500 ml three-necked flask equipped with a stirrer and reflux condenser, 1 g of agar agar and 0.5 g of Phenonip® (preservative mixture containing phenoxyethanol and parabens) were dissolved in 30 ml water in boiling heat. Then a homogeneous dispersion of 25 g glycerol, 10 g bamboo extract, 0.3 g White Tea extract, 5 g of a pigment mixture consisting of titanium dioxide and mica (1:1), and 0.5 g sodium alginate in 28 g water was added to the mixture over a period of about 30 min. under vigorous stirring. The matrix thus obtained was adjusted to a temperature of 50 °C and dispersed under vigorous stirring into a 2.5fold volume of paraffin oil having a temperature of about 15 °C. Subsequently the beads that were formed were filtered and added to a 0.5% by weight chitosan solution (Hydagen® DCMF, Cognis Deutschland GmbH & Co. KG, Düsseldorf/FRG). To obtain microcapsules of the same diameter, the preparations were then sieved and finally mixed with preserved water for conservation.

### Example 2

A mixture consisting of 1g agar agar, 0.5 g Phenonip, 25 g glycerol, 10 g bamboo extract, 2 g Citrus Aurantium extract, 5 g of a pigment mixture of iron oxide and mica (1:1), and 0.2 g sodium alginate in 28 g water was dropped by means of a Mini-Droppo equipment (Rieter Automatik GmbH, FRG; diameter of the nozzle: 0.8 mm) into a bath comprising an aqueous solution of calcium chloride (1 % b.w.) and chitosan (0.1 % b.w.). After filtration, the beads were sieved and finally mixed with preserved water for conservation.

The following Table 1 discloses a couple of skin care compositions comprising the capsules obtained according to Examples 1 and 2.

**Table 1**

| **Skin care compositions (water, preservatives add to 100 % b.w.)** | | | | | |
|---|---|---|---|---|---|
| **Composition (INCI)** | **A** | **B** | **C** | **D** | **E** |
| **Emulgade® SE** Glyceryl Sterate (and) Ceteareth 12/20 (and) Cetearyl Alcohol (and) Cetyl Palmitate | 5,0 | 5,0 | 4,0 | - | - |
| **Eumulgin® B1** Ceteareth-12 | - | - | 1,0 | - | - |
| **Lameform® TGI** Polyglyceryl-3 Isostearate | - | - | - | 4,0 | - |
| **Dehymuls® PGPH** Polyglyceryl-2 Dipolyhydroxystearate | - | - | - | - | 4,0 |
| **Monomuls® 90-O 18** Glyceryl Oleate | - | - | - | 2,0 | - |
| **Cetiol® HE** PEG-7 Glyceryl Cocoate | - | - | - | - | 2,0 |
| **Cetiol® OE** Dicaprylyl Ether | - | - | - | 5,0 | 6,0 |
| **Cetiol® PGL** Hexyldecanol (and) Hexyldecyl Laurate | - | - | 3,0 | 10,0 | 9,0 |
| **Cetiol® SN** Cetearyl Isononanoate | 3,0 | 3,0 | - | - | - |
| **Cetiol® V** Decyl Oleate | 3,0 | 3,0 | - | - | - |
| **Myritol® 318** Coco Caprylate Caprate | - | - | 3,0 | 5,0 | 5,0 |
| **Bees Wax** | - | - | - | 7,0 | 5,0 |
| **Nutrilan® Elastin E20** Hydrolyzed Elastin | 2,0 | - | - | - | - |
| **Nutrilan® I-50** Hydrolyzed Collagen | - | 2,0 | - | - | - |
| **Gluadin® AGP** Hydrolyzed Wheat Gluten | - | - | 0,5 | - | - |
| **Gluadin® WK** Sodium Cocoyl Hydrolyzed Wheat Protein | - | - | - | 0,5 | 0,5 |
| **Microcapsules, according to Example 1** | 1,0 | 1,0 | - | 1,0 | 1,0 |
| **Microcapsules, according to Example 2** | - | - | 1,0 | - | - |
| **Hydagen® CMF** Chitosan | 1,0 | 1,0 | 1,0 | 1,0 | 1,0 |
| **Magnesium Sulfate Hepta Hydrate** | - | - | - | 1,0 | 1,0 |
| **Glycerin** (86 Gew.-%ig) | 3,0 | 3,0 | 5,0 | 5,0 | 3,0 |

| | | | | | |
|---|---|---|---|---|---|
| (A) Soft creme, (B,C) Moisturizing emulsion, (D,E) Night creme | | | | | |

## Claims

1. A microcapsule having a mean diameter of from 0.1 to 5 mm, a membrane and a matrix containing at least one active principle, **characterised in that** said active principle is an exfoliating agent.

2. A microcapsule according to claim 1, **characterised in that** said active principle is a mixture of
(a) exfoliating agents, and
(b1) plant extracts and/or
(b2) pigments and/or
(b3) vitamins.

3. A microcapsule according to claim 1 or 2, **characterised in that** said exfoliating agents (component a) are silicates or plant extracts rich in silicates.

4. A microcapsule according to any of the preceding Claims 2 to 3, **characterised in that** said plant extract is an extract of bamboo.

5. A microcapsule according to any of the preceding Claims 2 to 4, **characterised in that** said plant extracts (component b1) are selected from the group consisting of *Ginkgo biloba, Oleacea europensis, Camilia sinensis* (Green Tea or White Tea) *Glyzyrrhiza glabra, Vaccinium myrtillus, Trifolium pratense, Litchi sinensis, Vitis, vinifera, Brassica oleracea, Punica granatum, Petroselinium crispum, Centella asiatica, Passiflora incarnata, Medicago sativa, Voleriano officinalis, Castanea sativa, Salix alba, Hapagophytum procumben, Aloe Barbadensis, Melissa officinalis,* and *Citrus aurantium.*

6. A microcapsule according to any of the preceding Claims 2 to 5, **characterised in that** said pigments (component b2) are selected from the group consisting of chlorophyll, titanium dioxide, zinc oxide, iron oxide, chromium oxides, chromium hydroxides, ultramarines, indigo pigments, manganese ammonium pyrophosphates, copper phtalocyanines and mica.

7. A microcapsule according to any of the preceding Claims 2 to 6, **characterised in that** said vitamins (component b3) are carotinoids and/or ascorbic acid.

8. A microcapsule according to any of the preceding Claims 1 to 7, **characterised in that** said active principles are present in amounts from 0.01 to 25 % b.w.

9. A microcapsule according to any of the preceding Claims 2 to 8, **characterised in that** the relation by weight between component (a) and component (b) is from about 10 : 90 to about 99 : 1%.

10. A microcapsule according to any of the preceding Claims 2 to 9, **characterised in that** the relation by weight between component (b1) on one hand and component (b2) and/or (b3) on the other is from about 99 : 1 to about 50 : 50.

11. A microcapsule according to any of the preceding Claims 1 to 10, **characterised in that** the shell is formed by coazervation, precipitation or polycondensation of an anionic and a cationic monomer or polymer.

12. A microcapsule according to any of the preceding Claims 1 to 11, **characterised in that** said anionic polymers are selected from alginates, carboxymethylcelluloses, gellan gum or acrylates.

13. A microcapsule according to any of the preceding Claims 1 to 12, **characterised in that** said cationic polymer are selected from chitosan, polyquaternium or gelatine.

14. A cosmetic composition comprising microcapsules according to claim 1.

15. Use of microcapsules according to claim 1 for making cosmetic compositions.

## Patentansprüche

1. Mikrokapsel mit einem mittleren Durchmesser von 0,1 bis 5 mm, einer Membran und einer Matrix, die mindestens einen Wirkstoff umfasst, **dadurch gekennzeichnet, dass** es sich bei dem Wirkstoff um eine Peeling-Substanz handelt.

2. Mikrokapsel nach Anspruch 1, **dadurch gekennzeichnet, dass** es sich bei dem Wirkstoff um eine Mischung aus
(a) Peeling-Substanzen und
(b1) Pflanzenextrakten und/oder
(b2) Pigmenten und/oder
(b3) Vitaminen
handelt.

3. Mikrokapsel nach Anspruch 1 oder 2, **dadurch gekennzeichnet, dass** es sich bei den Peeling-Substanzen (Komponente a) um Silikate oder silikatreiche Pflanzenextrakte handelt.

4. Mikrokapsel nach einem der vorhergehenden Ansprüche 2 bis 3, **dadurch gekennzeichnet, dass** es sich bei dem Pflanzenextrakt um einen Bambusextrakt handelt.

5. Mikrokapsel nach einem der vorhergehenden Ansprüche 2 bis 4, **dadurch gekennzeichnet, dass** die Pflanzenextrakte (Komponente b1) aus der Gruppe *Ginkgo biloba, Oleacea europensis, Camilia sinensis* (Grüntee oder Weißtee) *Glyzyrrhiza glabra, Vaccinium myrtillus, Trifolium pratense, Litchi sinensis, Vitis, vinifera, Brassica oleracea, Punica granatum, Petroselinium crispum, Centella asiatica, Passiflora incarnata, Medicago sativa, Valeriana officinalis, Castanea sativa, Salix alba, Hapagophytum procumben, Aloe Barbadensis, Melissa officinalis* und *Citrus aurantium* stammen.

6. Mikrokapsel nach einem der vorhergehenden Ansprüche 2 bis 5, **dadurch gekennzeichnet, dass** die Pigmente (Komponente b2) aus der Gruppe Chlorophyll, Titandioxid, Zinkoxid, Eisenoxid, Chromoxide, Chromhydroxide, Ultramarin-Verbindungen, Indigopigmente, Manganammoniumpyrophosphate, Kupferphthalocyanine und Glimmer stammen.

7. Mikrokapsel nach einem der vorhergehenden Ansprüche 2 bis 6, **dadurch gekennzeichnet, dass** es sich bei den Vitaminen (Komponente b3) um Carotinoide und/oder Ascorbinsäure handelt.

8. Mikrokapsel nach einem der vorhergehenden Ansprüche 1 bis 7, **dadurch gekennzeichnet, dass** die Wirkstoffe in Mengen von 0,01 bis 25 Gew.-% vorliegen.

9. Mikrokapsel nach einem der vorhergehenden Ansprüche 2 bis 8, **dadurch gekennzeichnet, dass** das Gewichtsverhältnis zwischen Komponente (a) und (b) von ungefähr 10:90 bis ungefähr 99:1 beträgt.

10. Mikrokapsel nach einem der vorhergehenden Ansprüche 2 bis 9, **dadurch gekennzeichnet, dass** das Gewichtsverhältnis zwischen Komponente (b1) einerseits und Komponente (b2) und/oder (b3) andererseits von ungefähr 99:1 bis ungefähr 50:50 beträgt.

11. Mikrokapsel nach einem der vorhergehenden Ansprüche 1 bis 10, **dadurch gekennzeichnet, dass** die Hülle durch Koazervation, Fällung oder Polykondensation eines anionischen und eines kationischen Monomers oder Polymers entsteht.

12. Mikrokapsel nach einem der vorhergehenden Ansprüche 1 bis 11, **dadurch gekennzeichnet, dass** die anionischen Polymere aus der Reihe Alginate, Carboxymethylcellulosen, Gellan-Gummi oder Acrylate stammen.

13. Mikrokapsel nach einem der vorhergehenden Ansprüche 1 bis 12, **dadurch gekennzeichnet, dass** die kationischen Polymere aus der Reihe Chitosan, Polyquaternium oder Gelatin stammen.

14. Kosmetikzusammensetzung, umfassend Mikrokapseln nach Anspruch 1.

15. Verwendung von Mikrokapseln nach Anspruch 1 für die Herstellung von Kosmetikzusammensetzungen.

## Revendications

1. Microcapsule ayant un diamètre moyen allant de 0,1 à 5 mm, une membrane et une matrice contenant au moins un principe actif, **caractérisée en ce que** ledit principe actif est un agent exfoliant.

2. Microcapsule selon la revendication 1, **caractérisée en ce que** ledit principe actif est un mélange :
(a) d'agents exfoliants, et
(b1) d'extraits de plantes et/ou
(b2) de pigments et/ou
(b3) de vitamines.

3. Microcapsule selon la revendication 1 ou 2, **caractérisée en ce que** lesdits agents exfoliants (composant a) sont des silicates ou des extraits de plantes riches en silicates.

4. Microcapsule selon l'une quelconque des revendications 2 à 3 précédentes, **caractérisée en ce que** ledit extrait de plantes est un extrait de bambou.

5. Microcapsule selon l'une quelconque des revendications 2 à 4 précédentes, **caractérisée en ce que** lesdits extraits de plantes (composant b1) sont choisis dans le groupe constitué par *Ginkgo biloba, Oleacea europensis, Camilia sinensis* (thé vert ou thé blanc) *Glyzyrrhiza glabra, Vaccinium myrtillus*, *Trifolium pratense, Litchi sinensis, Vitis vinifera, Brassica oleracea, Punica granatum, Petroselinium crispum, Centella asiatica, Passiflora incarnata, Medicago sativa, Valeriana officinalis, Castanea sativa, Salix alba, Harpagophytum procumben, Aloe barbadensis, Melissa officinalis* et *Citrus aurantium.*

6. Microcapsule selon l'une quelconque des revendications 2 à 5 précédentes, **caractérisée en ce que** lesdits pigments (composant b2) sont choisis dans le groupe constitué par la chlorophylle, le dioxyde de titane, l'oxyde de zinc, l'oxyde de fer, les oxydes de chrome, les hydroxydes de chrome, les outremers, les pigments à base d'indigo, les pyrophosphates de manganèse et d'ammonium, les phtalocyanines de cuivre et le mica.

7. Microcapsule selon l'une quelconque des revendications 2 à 6 précédentes, **caractérisée en ce que** lesdites vitamines (composant b3) sont des caroténoïdes et/ou de l'acide ascorbique.

8. Microcapsule selon l'une quelconque des revendications 1 à 7 précédentes, **caractérisée en ce que** lesdits principes actifs sont présents selon des quantités allant de 0,01 à 25% en poids.

9. Microcapsule selon l'une quelconque des revendications 2 à 8 précédentes, **caractérisée en ce que** la relation en poids entre le composant (a) et le composant (b) va d'environ 10:90 à environ 99:1%.

10. Microcapsule selon l'une quelconque des revendications 2 à 9 précédentes, **caractérisée en ce que** la relation en poids entre le composant (b1) d'une part et le composant (b2) et/ou (b3) d'autre part va d'environ 99:1 à environ 50:50.

11. Microcapsule selon l'une quelconque des revendications 1 à 10 précédentes, **caractérisée en ce que** l'enveloppe est formée par coacervation, précipitation ou polycondensation d'un monomère ou polymère anionique et cationique.

12. Microcapsule selon l'une quelconque des revendications 1 à 11 précédentes, **caractérisée en ce que** lesdits polymères anioniques sont choisis parmi les alginates, les carboxyméthylcelluloses, la gomme gellane ou les acrylates.

13. Microcapsule selon l'une quelconque des revendications 1 à 12 précédentes, **caractérisée en ce que** lesdits polymères cationiques sont choisis parmi le chitosane, le polyquaternium ou la gélatine.

14. Composition cosmétique comprenant des microcapsules selon la revendication 1.

15. Utilisation de microcapsules selon la revendication 1, pour la préparation de compositions cosmétiques.
